# EUROPÄISCHE PATENTSCHRIFT

(11) **EP 3 107 493 B1**
(45) Veröffentlichungstag und Bekanntmachung des Hinweises auf die Patenterteilung: **26.12.2018**
(21) Anmeldenummer: 15711648.4
(22) Anmeldetag: 13.02.2015
(51) Int. Cl.: A61F 2/20, A61M 16/04, A61M 16/10

(54) **SPRECHVENTIL MIT ZUMINDEST TEILWEISE AUS EINEM ELASTISCHEN MATERIAL GEBILDETEN DECKELTEIL**
SPEAKING VALVE HAVING A COVER PART FORMED AT LEAST PARTIALLY OF AN ELASTIC MATERIAL
VALVE DE PHONATION DOTÉE D'UNE PARTIE COUVERCLE RÉALISÉE AU MOINS EN PARTIE DANS UN MATÉRIAU ÉLASTIQUE

(30) Priorität: 18.02.2014 DE 102014002063
(43) Veröffentlichungstag der Anmeldung: 28.12.2016
(73) Patentinhaber: Andreas Fahl Medizintechnik-Vertrieb GmbH, 51149 Köln (DE)
(72) Erfinder: FAHL, Andreas, 50676 Köln (DE)
(74) Vertreter: Geskes, Christoph
(86) Internationale Anmeldenummer: PCT/EP2015/000315
(87) Internationale Veröffentlichungsnummer: WO 2015/124278

(56) Entgegenhaltungen:
- EP-A1- 2 236 165
- WO-A1-99/60954
- WO-A1-2011/144237
- DE-U1-202013 008 092

## Beschreibung

Die Erfindung betrifft ein Sprechventil für Laryngektomierte oder Tracheotomierte mit einem Deckelteil, einem Stellmittel, einem Verschlussteil, einem Gehäuseteil und einem im Gehäuse angeordneten Ventilsitz.

Sprechventile sind aus dem Stand der Technik bekannt. Diese werden z.B. an eine Tracheostomakanüle oder ein Tracheostomapflaster angebracht, damit Menschen ohne Stimmbänder mittels einer Stimmprothese sprechen können. Durch Betätigen des Sprechventils wird Luft durch eine Stimmprothese, die in einer Fistel zwischen Trachea und Ösophagus angeordnet ist, geleitet. Auch wenn die Stimmbänder noch vorhanden sind ist es notwendig, dass die Luft nicht aus der Trachealkanüle entweicht, bevor diese die Stimmbänder erreicht.

Aus der US 4,582,058A ist bekannt, ein Sprechventil mittels eines Druckimpulses, der durch die Atmung des Patienten erfolgt, zu schließen und dadurch ein Sprechen zu ermöglichen. Jedoch haben sich diese Ventile als nicht besonders zuverlässig erwiesen.

Die WO 95/17138 A schlägt vor, das Sprechventil mit einer künstlichen Nase zu kombinieren. Diese künstliche Nase ist ein Filter, der Feuchtigkeit und Wärme der ausgeatmeten Luft auffängt und die in die Trachealkanüle einströmende Luft anfeuchtet und erwärmt.

Die EP 1 077 658 B1 beschreibt ein Stimmventil mit einem Filter, wobei das Stimmventil ein elastisches Gehäuse bzw. elastisches Ventilelement aufweist und das Gehäuse bzw. Ventilelement derart beispielsweise mittels Fingerdruck verformt werden kann, dass ein Gehäuseteil zur Auflage auf einem Ventilsitz kommt und das Ventil damit geschlossen wird.

DE 20 2013 008 092 U1 beschreibt einen Wärmefeuchtigkeitsaustauscher ohne Gehäuse mit Befeuchtungs- und Sprechfunktion für Laryngektomierte oder Tracheotomierte. Dieser ist derart ausgestaltet, dass der Filter eine Stabilität des Wärmefeuchtigkeitsaustauscher gewährleistet, wobei der Filter und weitere luftdurchlässige, vornehmlich aus Schaumstoff bestehenden Teile bei Betätigung des Sprechventils zusammengequetscht werden. WO99/60954 offenbart Sprechventil mit einem Filter.

Aufgabe der vorliegenden Erfindung ist es, ein Sprechventil zur Verfügung zu stellen, welches einfach aufgebaut und kostengünstig herzustellen ist.

Diese Aufgabe wird erfindungsgemäß gelöst mittels eines Sprechventils nach Anspruch 1. Weitere vorteilhafte Ausgestaltung sind der nachfolgenden Beschreibung, den Figuren sowie den Unteransprüchen zu entnehmen. Die einzelnen Merkmale der beschriebenen Ausgestaltung sind jedoch nicht auf diese beschränkt, sondern können untereinander und mit anderen Merkmalen zu weiteren Ausgestaltungen verknüpft werden.

Es wird ein Sprechventil für Laryngektomierte oder Tracheotomierte vorgeschlagen, wobei das Sprechventil ein Deckelteil, ein Stellmittel, ein Gehäuseteil und einen Filter aufweist. Das Gehäuseteil stellt einen proximal in diesem angeordneten Ventilsitz zur Verfügung. Das Stellmittel umfasst ein Verschlussteil, wobei das Verschlussteil bei Überführung des Sprechventiles aus einer Offenstellung in eine Geschlossenstellung zur Ermöglichung eines Sprechens mit dem Ventilsitz zusammenwirkt. Das Deckelteil ist zumindest teilweise aus einem elastischen Material gebildet. Bei einer Überführung des Sprechventiles aus der Offenstellung in die Geschlossenstellung erfolgt eine zumindest teilweise Verformung zumindest eines Bereiches des Deckelteiles. Der Filter ist derart am Stellmittel angeordnet, dass dieser im Wesentlichen der Bewegung des Stellmittels folgen kann, und bevorzugt folgt. Der proximal angeordnete Ventilsitz ist körpernah bzw. körpernäher ausgebildet als eine distale Öffnung des Sprechventils, welche sich oberhalb des proximal angeordneten Ventilsitzes befindet. Dabei kann der proximal angeordnete Ventilsitz z. B. durch eine Bodenplatte, ausgeführt als Ring, gebildet sein, oder aber durch eine bevorzugt ringförmige Fläche, die an der Gehäusewandung zur Verfügung gestellt ist. Dabei kann auch vorgesehen sein, dass eine proximale Öffnung zur Verfügung gestellt ist durch in das Innere des Gehäuses ragende Wandteile, ausgehend von einem Bodenring, um ein Aufsetzen des Sprechventils auf eine 15 mm-Tracheal-Kanüle zu ermöglichen. Die Wandteile können dabei z.B. als sockelähnlich beschrieben werden. Derartige Kombinationsventile können z.B. gemäß der DE 10 2005 007 234 B3 ausgebildet sein. Der Ventilsitz wird dabei nicht durch die genannten Wandteile gebildet. Die Rückstellkräfte, die bei einer Überführung des Sprechventils von einer zum Sprechen notwendigen Geschlossenstellung in eine Offenstellung benötigt werden, werden überwiegend, im wesentlichen vollständig von dem aus einem elastischen Material gebildeten Deckelteil zur Verfügung gestellt. Dadurch entfallen vorteilhafterweise ansonsten notwendige Rückstellmittel wie Federn oder ähnliches. Da durch die Mitführung des Filters dieser allenfalls gering, bevorzugt im wesentlichen nicht komprimiert wird, bleibt dessen Funktion des Wärme- und Feuchtigkeitsaustausches so gut wie vollständig, wenn nicht vollständig erhalten.

Unter dem Begriff "distal" wird im Sinne der vorliegenden Erfindung in Bezug auf ein Merkmal der erfindungsgemäßen Vorrichtung eine Anordnung oder Verwendung desselben fern oder auch abgewandt oder gegenüberliegend eines Tracheostomapflasters oder einer Trachealkanüle, allgemeiner einer Hautoberfläche einer Person, die insbesondere solche Befestigungsmittel für die erfindungsgemäße Vorrichtung trägt, verstanden. Unter dem Begriff "proximal" wird im Sinne der vorliegenden Erfindung in Bezug auf ein Merkmal der erfindungsgemäßen Vorrichtung eine Anordnung oder Verwendung desselben nah oder auch zugewandt oder benachbart eines Tracheostomapflasters oder einer Trachealkanüle, allgemeiner einer Hautoberfläche einer Person, die insbesondere solche Befestigungsmittel für die erfindungsgemäße Vorrichtung trägt, verstanden.

Soweit in der vorliegenden Erfindung Richtungsangaben genannt sind, so sind diese immer in Bezug auf den bestimmungsgemäßen Einbau des Sprechventiles am Körper zu verstehen. Bestimmungsgemäß wird das erfindungsgemäße Sprechventil über einem Tracheostoma platziert, und insbesondere an einer Trachealkanüle oder einem Tracheostomapflaster befestigt.

Soweit in der vorliegenden Erfindung der Begriff "im Wesentlichen" verwendet wird, gibt dieser im Sinne der Erfindung einen Toleranzbereich an, der für den Fachmann unter wirtschaftlichen und technischen Gesichtspunkten zu vertreten ist, so dass das entsprechende Merkmal noch als solches zu erkennen ist und die diesem zukommende Funktion erfüllt.

Das Deckelteil ist vorteilhafterweise hut- oder kappenmäßig ausgebildet. Hierunter ist eine Ausgestaltung des Deckelteiles derart zu verstehen, dass das Deckelteil eine Seitenwandung des Gehäuseteiles zumindest teilweise, bevorzugt vollständig (d.h. nach allen Seiten) überragt. Das Überragen der Seitenwandung des Gehäuseteils erfolgt dabei vorteilhafterweise in einer Richtung im Wesentlichen parallel zu einer Hautoberfläche des Körpers. Weiter bevorzugt erfolgt die Überragung der Seitenwandung des Gehäuseteils vertikal, d.h. in Richtung der vertikalen Erstreckung des Gehäuseteiles von seinem proximalen Ende zu seinem distalen Ende. Das vertikale Überragen erfolgt bevorzugt über eine Teillänge der vertikalen Erstreckung des Gehäuseteils. Das Deckelteil kann in dem die Seitenwandung des Gehäuseteils überragenden Bereich auch Öffnungen, insbesondere schlitzförmig ausgebildete, aufweisen.

Vorteilhafterweise weist das Deckelteil von proximal nach distal, bezogen auf die Endbereiche des Gehäuseteils, eine Höhe auf, die mindestens 30 % bis etwa 95 %, bevorzugt etwa 40 % bis etwa 95 %, weiter bevorzugt etwa 50 % bis etwa 95 %, noch weiter bevorzugt etwa 70 % bis etwa 95 %, noch weiter bevorzugt etwa 80 % bis etwa 90 % einer Höhe des Gehäuseteils entspricht. Bevorzugt weist das Deckelteil von proximal nach distal eine Höhe auf, die maximal etwa 95 %, weiter bevorzugt maximal etwa 90 %, noch weiter bevorzugt maximal etwa 80 % der Höhe des Gehäuseteils, ermittelt ausgehend von der proximalen Unterkante, die dem Körper zugewandt ist, bis zur distalen Oberkante, die vom Körper weg zeigt, der Seitenwandung derselben.

Die Höhe des Deckelteils von proximal nach distal wird bestimmt einerseits durch den zervikalen Rand, andererseits durch die distale Oberfläche desselben, die am weitesten vom Tracheostoma absteht.

Soweit im Rahmen der vorliegenden Erfindung der Begriff "etwa" verwendet wird, so ist darunter ein Toleranzbereich zu verstehen, den der angesprochene Fachmann auf dem vorliegenden Gebiet für üblich erachtet, insbesondere ein Toleranzbereich von +/- 20 %, bevorzugt +/- 10 %, weiter bevorzugt +/- 5 %, jeweils bezogen auf den in Rede stehenden Wert, bezogen auf die angegebenen Werte.

In einer bevorzugten Ausführungsform überragt das Deckelteil das Gehäuseteil sowohl lateral als auch vertikal. Weiter bevorzugt umfasst das Deckelteil einen zervikalen Rand. Der zervikale Rand, der dem Hals eines Trägers des erfindungsgemäßen Sprechventiles zugewandt ist, ist vorzugsweise abgerundet ausgebildet, so dass keine Hautirritationen auftreten, wenn bei Bewegung des Benutzers ein Hautkontakt erfolgte. Insbesondere weist das Deckelteil bei lateraler Draufsicht eine runde, eine ovale oder sonstwie gerundete oder eine rechteckige Formgebung auf, wobei bei einer eckigen Formgebung die Ecken vorteilhafterweise abgerundet sind.

Vorteil eines jeden der Merkmale, der vorstehend im Zusammenhang mit der Ausgestaltung des Deckelteiles beschrieben sind, ist eine Führung der Luft beim Einatmen am Hals entlang, so dass die Luft, bevor sie das Innere des Sprechventiles und insbesondere den Filter erreicht, vorgewärmt ist. Je nach Ausgestaltung des Deckelteiles, insbesondere im Hinblick auf die Anordnung des zervikalen Randes eher nah an der Seitenwandung des Gehäuseteiles oder eher entfernt von dieser, als auch durch die Anordnung des Deckelteiles mit dem zervikalen Rand derart, dass der zervikale Rand nahe oder etwas weiter beabstandet dem Hals des Benutzers zugeordnet ist, kann sowohl das Volumen des aufzunehmenden Luftstromes als auch die Vorwärmung desselben eingestellt werden.

Besonders vorteilhaft ist es dabei, wenn das Deckelteil distal, und dort insbesondere in einem zentralen, dem Gehäuseteil zugewandten Bereich, keine Öffnung aufweist, die einen Luftdurchtritt von distal nach proximal durch den Filter hindurch ermöglicht. Das Gehäuseteil umfasst dann vorzugsweise eine oder mehrere Einströmöffnungen in einer Seitenwandung desselben. Hierdurch wird sichergestellt, dass die Luft im Wesentlichen am Hals entlanggeführt wird, bevor sie in das Gehäuseteil eintritt. Alternativ oder zusätzlich kann jedoch auch vorgesehen sein, dass das Deckelteil mindestens eine distale Öffnung, insbesondere in einem Bereich, der dem Inneren des Gehäuseteils zugewandt ist, aufweist. Durch die mindestens eine distale Öffnung kann zumindest ein Teil der eingeatmeten Luft direkt in das Gehäuse bzw. durch das Filter dringen, so dass die Luft erfeuchtet und erwärmt in die Trachea bzw. in die Trachealkanüle eindringen kann.

Vorzugsweise ist das Deckelteil lösbar mit dem Gehäuseteil und/oder dem Stellmittel verbunden. Bevorzugt ist das Deckelteil lösbar mit dem Gehäuseteil und fest mit dem Stellmittel verbunden. In einer weiter bevorzugten Ausführungsform ist das Deckelteil lösbar mit dem Gehäuseteil und lösbar mit dem Stellmittel verbunden. Die Lösbarkeit insbesondere des Stellmittels ist vorzugsweise erschwert im Vergleich zu derjenigen des Deckelteils. Bei den letztgenannten Ausführungsformen stellt das Deckelteil mit dem Stellmittel und dem an diesem angeordneten Filter ein Bauteil des erfindungsgemäßen Sprechventiles dar, das andere Bauteil wird durch das Gehäuseteil gebildet. Das Deckelteil kann einstückig mit dem Stellmittel ausgebildet sein. Vorteilhafterweise ist das Deckelteil mittels einer Rastverbindung an dem Gehäuseteil angeordnet. Aber auch andere Möglichkeiten einer lösbaren Verbindung, beispielsweise zur Verfügung gestellt durch eine oder mehrere Klettverbindungen oder Klebeverbindungen, sind möglich. Die Rastverbindung kann derart ausgeführt sein, dass das Deckelteil zwei, drei, vier oder mehr Rastelemente, beispielsweise in Form von Rastnasen, aufweist, die an einer Innenfläche des Deckelteiles, der Seitenwandung des Gehäuseteiles zugeordnet, angeordnet sind. Dabei kann vorgesehen sein, dass die Rastelemente einen oberen Rand des Gehäuseteiles umfassen in einem Bereich desselben, in dem Einströmöffnungen vorgesehen sind. An diesem Rand können entsprechende Aufnahmen oder Ausnehmungen oder aber auch Durchbrüche vorgesehen sein, in welche die Rastelemente des Deckelteiles einrasten. Umgekehrt können entsprechende Rastelemente auch im distalen Endbereich des Gehäuseteiles angeordnet sein, beispielsweise Rastnasen, die in entsprechende Ausnehmungen, angeordnet an der Unterseite des Deckelteiles, einrasten. Die lösbare Verbindung zwischen Deckelteil und Gehäuseteil ist vorzugsweise derart ausgestaltet, dass im Verbindungsbereich keine Luft zwischen Deckelteil und Gehäuseteil hindurch ein- oder ausströmen kann. Es kann beispielsweise vorgesehen sein, dass an der Innenseite des Deckelteils drei oder vier Rastnasen und/oder Ausnehmungen angeordnet sind, welche wiederum auf einem zur Erzielung einer hinreichend festen Verbindung auf der Innenseite des Deckelteiles angeordneten Bund oder Wulst angeordnet sind, der in seiner Dimensionierung, d.h. insbesondere in seinem Durchmesser, dem Durchmesser des Gehäuseteils entspricht, und den oberen Rand der Seitenwandung des Gehäuseteils in etwa ab- oder überdeckt. Die Rastelemente bzw. -nasen greifen bevorzugt in entsprechende Ausnehmungen am oberen Rand des Deckelteils ein, in die Ausnehmungen können umgekehrt Rastnasen am oberen Rand des Deckelteils eingreifen. Der Wulst oder Bund kann dabei nur gering beabstandet vom zervikalen Rand des Deckelteils angeordnet sein, soweit das Deckelteil nur die Seitenwandung des Gehäuseteils überragt. Das Deckelteil kann aber beispielsweise mit dem Gehäuseteil vernietet oder verschraubt sein, oder aber auch beispielsweise in Form einer Bajonettverschlusses mit dem Gehäuseteil verbunden sein.

Erfindungsgemäß ist das Deckelteil zumindest teilweise aus einem elastischen Material gebildet. Vorteilhafterweise ist das elastische Material des Deckelteils ein gummielastisches Material. Bevorzugt ist das elastische Material des Deckelteiles ein linear-elastisches Material. Als gummielastisches Material kann insbesondere Naturkautschuk oder aber Synthesekautschuk, insbesondere Butyl-Kautschuk, oder aber Ethylen-Propylen-Dien-Kautschuk (EPDM), eingesetzt werden. Aber auch der Einsatz von Silikonmaterialien, insbesondere medizinischen Silikonmaterialien, ist möglich. Grundsätzlich kann das gummielastische Material des Deckelteiles ein Elastomer sein, beispielsweise ein thermoelastisches Elastomer auf Olefin- oder Urethanbasis, besonders bevorzugt ein zumindest teilweise vernetztes thermoplastisches Elastomer auf Olefinbasis, ein Polyesterelastomer, ein thermoplastischer Copolyester, ein Styrolblock-Polymere oder ein thermoplastisches Copolymere. Anstatt aus einem gummielastischen Material hergestellt zu sein, welches aus einem Polymer gebildet ist, kann das Deckelteil zumindest teilweise aus einem linear-elastischem Material, insbesondere einem Metall, gebildet sein, welches eine hinreichende Elastizität aufweist. Aber auch andere Materialien, die eine hinreichende Elastizität zur Verfügung stellen, sind möglich. Besonders bevorzugt besteht das Deckelteil aus einem elastischen, bevorzugt gummielastischem Material, weist mithin kein weiteres Material auf. Das Deckelteil kann dabei eine gleichmäßige Stärke aufweisen, die Stärke kann jedoch insbesondere im Bereich des Überganges von einer horizontalen in eine vertikale Erstreckung des Deckelteiles verringert sein, und/oder ebenso in einem Bereich, durch welchen durch die Benutzer mittels eines Fingers eine Überführung des Sprechventiles aus der Offenstellung in die Geschlossenstellung und zurück erfolgt.

In einer bevorzugten Ausführungsform fungiert zumindest ein elastisch verformbarer Bereich des Deckelteiles als Rückstellmittel zur Überführung aus der Geschlossenstellung in die Offenstellung. Durch die Elastizität des Deckelteiles wird dabei letztendlich nach Betätigen des Sprechventiles durch den Benutzer, indem über das Deckelteil eine Kraft in Richtung auf das Tracheostoma ausgeübt und dabei das Verschlussteil in Wirkverbindung mit dem Ventilsitz zur Schließung des Sprechventiles gelangt, durch Entlastung, also Wegnahme beispielsweise des Fingers des Benutzers, der zur Schließung des Sprechventils benutzt wurde, eine Rückführung aus der Geschlossen- in die Offenstellung des Sprechventiles erzielt, so dass dann ein Atmen durch das Sprechventil wieder ermöglich ist. Diese Funktion wird durch das Deckelteil im Ganzen oder aber durch einen Teilbereich desselben erfüllt. Das Deckelteil kann beispielsweise auch derart ausgebildet sein, dass es einen elastischen Bereich dort aufweist, wo das Deckelteil dem Inneren des Gehäuseteiles zugewandt ist, welchen sich dann beispielsweise ein weniger elastischer Bereich, oder aber gar ein unelastischer Bereich anschließt, in welchem dann beispielsweise die vorstehend angesprochenen Rastelemente auf der Innenwandung des Deckelteiles angeordnet sind. Bei Überragen der Außenfläche der Seitenwandung des Gehäuseteiles, wenn also insbesondere das Deckelteil in Form eine Hutes oder eines Kappe ausgebildet ist, kann dabei dieser überragende Bereich auch aus einem hinreichend festen Material hergestellt sein. Dann kann beispielsweise der zervikale Rand oder aber Randbereich wiederum aus einem weicheren Material, beispielsweise Silikon, hergestellt sein, um Hautirritationen zu vermeiden. Auch kann beispielsweise vorgesehen sein, dass in dem dem Inneren des Gehäuseteiles zugewandten Bereich das Deckelteil aus einem linear-elastischen Material wie beispielsweise einem entsprechend geeigneten Metall ausgebildet ist, an welches sich dann bei Vorsehung einer Überragung der Außenseite der Außenwandung des Gehäuseteiles ein hinreichend flexibeles elastisches Kunststoffmaterial anschließt. Bei einer Ausgestaltung mit einem Deckteil mit zumindest einem elastischen Bereich, der dem Inneren des Gehäuseteils zugewandt ist, kann zur Befestigung des Stellmittels ein verstärkter Bereich in Form einer Materialanhäufung, insbesondere in Ringform, vorgesehen sein, in welche das Stellmittel über geeignete Befestigungsmittel einführgar zum Zwecke der Befestigung ist. Aber auch eine jedwede andere Ausgestaltung eines Deckelteiles sind denkbar, soweit dieses die Funktion erfüllt, als Rückstellmittel zur Überführung des erfindungsgemäßen Sprechventiles aus der Geschlossenstellung in die Offenstellung zu dienen.

Das Gehäuseteil umfasst vorteilhafterweise zumindest eine Einströmöffnung in der Seitenwandung desselben. Vorzugsweise können mehrere Einströmöffnungen, insbesondere drei, vier, fünf oder sechs oder aber mehr vorgesehen sein. Dabei kann dann das Gehäuseteil zumindest teilweise rahmenartig ausgestaltet werden. In dem rahmenartig ausgestalteten Bereich sind Einströmöffnungen angeordnet, wobei zwischen diesen im Wesentlichen nur Streben angeordnet sind, die einen unteren, proximalen Endbereich des Sprechventiles mit einem oberen Endbereich, insbesondere einem als umlaufenden Ring ausgebildeten, verbinden. Durch die Streben kann vorteilhafterweise dem Filter eine Stützfunktion mitgegeben werden. Auch wird hierdurch zumindest indirekt eine Führung des Stellmittels im Inneren des Gehäuseteils bei Überführung aus der Offenstellung in die Geschlossenstellung und umgekehrt zur Verfügung gestellt. Die Einströmöffnungen sind möglichst groß ausgebildet, bevorzugt nehmen sie etwa 30 % bis etwa 90 %, weiter bevorzugt etwa 40 % bis etwa 90 % noch weiter bevorzugt etwa 40 % bis etwa 85 % der Seitenwandung ein. Die Einströmöffnungen können jegliche Geometrie aufweisen, beispielsweise können die Einströmöffnungen auch siebartig ausgebildet sein. Durch eine zur Verfügungstellung einer möglichst großen Fläche für die Einströmöffnungen in der Seitenwandung des Gehäuseteiles wird ein möglichst großer Luftdurchtritt durch selbige erzielt.

An seinem proximalen Ende umfasst das Gehäuseteil vorteilhafterweise eine Öffnung zur Verbindung mit dem Tracheostoma. Diese Öffnung ist in einem Boden des Gehäuseteiles angeordnet. Der Boden wird bevorzugt gebildet durch einen umlaufenden, an der Seitenwandung ansetzenden ringförmigen Rand, wobei eine Verstärkung vorteilhafterweise durch Verstrebungen erfolgte, die kreuzartig innerhalb der von dem ringförmigen Rand des Bodens gebildeten Bereich angeordnet und mit dem ringförmigen Rand verbunden sind. Eine Oberfläche des ringförmigen Randes des Bodens, zumindest ein Teilbereich derselben, dient als Ventilsitz für das erfindungsgemäße Sprechventil, und kann in diesem Bereich beispielsweise eine Auflage aus einem geeigneten Material aufweisen, um eine möglichst gute Dichtwirkung zu erzielen. Beispielsweise kann in den entsprechenden Bereichen ein Medizinsilikon angeordnet sein, oder aber auch jedes andere geeignete Dichtmaterial. Auch kann bei Vorsehung eines Bodenkreuzes in Form von drei, vier, fünf oder mehr Streben im Kreuzungspunkt derselben ein Führungsmittel für das Stellmitte vorgesehen sein, dass in das Innere des Gehäuseteils ragt. Dieses Führungsmittel kann beispielsweise stabförmig ausgebildet sein. Es greift bevorzug in eine Ausnehmung des Stellmittels im Verschlußteil und/oder einem Verbindungsteil des Stellmittels ein, welches das Verschlußteil mit dem Deckelteil verbindet, um eine Bewegung des Stellmittels über das Decketeil zu ermöglichen. Die Ausnehmung kann beispielsweise durch ein zumindest einseitig proximal offenes, rohrförmiges Verbindungsmittel zur Verfügung gestellt sein, in die ein stabförmig ausgebildetes Führungsmittel eingreifbar ist.

Im distalen Endbereich des Gehäuseteiles können zur Vorsehung einer lösbaren Befestigung mit dem Deckelteil Rastnasen oder sonstige Rastelemente vorgesehen sein, welche insbesondere an einem umlaufenden Ring angeordnet sind. Dort können ebenso zusätzlich oder alternativ auch Ausnehmungen oder Durchbrüche vorgesehen sein, in welche Rastelemente, angeordnet an der Innenwandung des Deckelteiles, zugewandt dem Gehäuseteil, eingreifen können oder aufgenommen werden.

Vorteilhafterweise weist mindestens eine Einströmöffnung in der Seitenwandung des Gehäuseteiles eine laterale Ausdehnung bis zu einer Höhe des Stellmittels und/oder bis zu etwa einer Höhe des Filters auf. Vorteilhafterweise ist die laterale Ausdehnung der Einströmöffnung dabei, bezogen auf einem in Inneren des Gehäuseteiles am Stellmittel angeordneten Filter, derart gewählt, dass der Filter das proximale und/oder das distale Ende der Einströmöffnung, bevorzugt das proximale Ende, etwas überragt, um sicherzustellen, dass der eingeatmete Luftstrom möglichst vollständig durch den Filter geht.

Das distale Ende des Gehäuseteiles weist eine distale Öffnung auf, durch welche das Stellmittel mit dem Filterkörper in das Gehäuseinnere eingebracht werden kann. Dabei kann über diese distale Öffnung im Gehäuseteil ebenfalls ein Luftstrom geführt werden, soweit entsprechende Öffnungen im Stellmittel und/oder Deckelteil vorgesehen sind. Diese distalen Öffnungen im Stellmittel und/oder Deckelteil müssen selbstverständlich so beschaffen sein, dass diese bei Betätigung das Sprechventil durch den Benutzer, üblicherweise über einen Finger, luftdicht verschlossen werden können.

Das Gehäuseteil ist vorteilhafterweise aus einem rigiden, d.h. unelastischen bzw. biegensteifen Material gebildet, beispielsweise aus einem geeigneten Polyethylen oder Polypropylen, insbesondere einem high-density Polyethylen (HDPE).

Der Filter umfasst bevorzugt einen offenzelligen Schaumstoff, der insbesondere antimikrobiell, beispielsweise mit Silber o.ä., beschichtet ist. Auch kann der Filter zur Erhöhung der Feuchteaufnahmefähigkeit mit entsprechenden Materialien getränkt sein. Vorteilhafterweise umfasst bei einer Ausführung des Filters in Schaumstoffform dieser zumindest ein Material aus einer Gruppe umfassend Polyester, Polyethylen und/oder Polyurethan. Es kann aber auch jedes andere einsetzbare Material für den Filter vorgesehen sein, beispielsweise kann dieser auch aus Papier oder Metall gefertigt sein. Bevorzugt übt der Filter eine Wärmetauscher- und/oder Feuchtigkeitstauscherfunktion aus, wie diese auch im eingangs genannten Stand der Technik beschrieben ist. Vorzugsweise wird der Filter bei Betätigung des Sprechventils nicht oder nur geringfügig komprimiert
Das Stellmittel umfasst erfindungsgemäß das Verschlussteil, welches mit dem Ventilsitz, zur Verfügung gestellt durch das Gehäuseteil, zusammenwirkt. Am Stellmittel ist erfindungsgemäß der Filter derart angeordnet, dass dieser der Bewegung des Stellmittels im Wesentlichen folgen kann. Die Bewegung des Stellmittels erfolgt über Führung des Sprechventiles aus der Offenstellung in die Geschlossenstellung durch eine Betätigung des Benutzers, und auf Grund der Vorsehung eines elastischen Materiales im Deckelteil bei Rückstellung des Sprechventiles aus der Geschlossenstellung in die Offenstellung. Vorteilhafterweise bleibt sowohl bei der Überführung des Sprechventiles aus der Offenstellung in die Geschlossenstellung als auch aus der Geschlossenstellung in die Offenstellung der Filter im Wesentlichen unkomprimiert. Dies kann beispielsweise dadurch erfolgen, dass am distalen Ende des Stellmittels oberhalb des Filterkörpers ein Deckelteilbefestigungselement vorgesehen ist, welches im Wesentlichen plattenförmig ausgebildet ist. Dann kann der Filterkörper zwischen dem Verschlussteil und dem Deckelteil des Befestigungselements des Stellmittels angeordnet sein. Über den Bereich des Deckelteilbefestigungselementes kann dann letztendlich die Bewegung des Stellmittels erfolgen. Der Filter kann dabei mit dem Verschlussteil und dem Deckelteil und/oder Deckelteilbefestigungselement verklebt oder sonstwie verbunden sein. Das Stellmittel kann aber auch derart ausgebildet sein, dass zwischen dem Verschlussteil und dem Deckelteilbefestigungselement und/oder Deckelteil ein Verbindungsteil vorgesehen ist, welches stab- oder wandförmig ausgebildet ist, um eine hinreichende Festigkeit dem Stellmittel zu vermitteln. Im Querschnitt gesehen kann bei Vorsehung auch eines Deckelteilbefestigungselementes von einer in etwa einfach T-förmigen oder doppel-T-förmigen Ausgestaltung des Stellmittels gesprochen werden. Durch die Nichtkomprimierung des Filters, die durch die vorstehend geschilderten beispielhaften Ausgestaltung des Stellmittels ermöglicht ist, wird nicht nur eine Verletzung oder sonstige Beeinträchtigung der Funktion des Filters vermieden, sondern auch vermieden, dass in diesem aufgenommene Feuchtigkeit nicht in einer größeren Menge auf einmal ausgedrückt wird bei Betätigung des erfindungsgemäßen Sprechventiles. Das Verbindungsteil, welches das Verschlussteil und das Deckelteil, gegebenenfalls über das Deckelteilbefestigungsteilelement des Stellmittels, verbindet, kann dabei beispielsweise gebildet sein durch zwei sich kreuzende Wandteile, die Öffnungen aufweisen können, aber nicht müssen. In diesem Fall könnte dann, je nach Abmessungen der Wände, ein viergeteilter Filter am Stellmittel angeordnet sein. Ist das das Verbindungsteil beispielsweise rohr-, stab- oder stangenförmig ausgebildet, kann einfach ein einteiliger Filter mit einer Bohrung im Bereich des Verbindungsteiles eingesetzt werden. Ist das Verbindungsteil beispielsweise nur als eine Wand ausgebildet, die über den gesamten Durchmesser des Verschlussteils verläuft, so kann ein zweiteiliger Filter eingesetzt werden. Das Deckelteil kann auch mit einem Stellmittel nur mit Verschlussteil und Verbindungsteil verbunden sein. Beispielsweise ist dann der Verbindungsbereich zwischen Verbindungsteil und Deckelteil im Deckelteil aus einem festeren, bevorzugt nur gering elastischen oder unelastischen Material, gebildet. Die Verbindung von Stellmittel mit Deckteil über das Verbindungsmittel kann dabei lösbar oder unlösbar erfolgen. Bevorzugt ist eine lösbare Ausgestaltung, bei der die Lösung jedoch erschwert ist. Beispielsweise ist im Befestigungsbereich auf der Unterseite des Deckelteils zentral eine Materialanhäufung vorgesehen, beispielsweise in Form eines Ringes, in dessen mittige Öffnung das dem Verschlußteil entgegengesetzte Ende des Verbingungsteile mit einer Materialverbreiterung rastend anordbar ist. Deckelteil und Stellmittel mit Verschlußteil können auch einteilig, beispielsweise über eine Klebung über den dazwischen angeordneten Filter, ausgebildet sein. Es ist aber auch möglich, dass Stellmittel mit Verschlußteil und Deckelteil materialeinheitlich ausgebildet sind. Bevorzugt ist aber das Deckelteil zumindest teilweise, bevorzug vollständig, aus einem elastischen Material und das Stellmittel aus einem rigiden Material, wie dieses auch für das Gehäuseteil einsetzbar oder weiter unten beschrieben ist, gebildet.

Der Filter ist bevorzugt derart ausgebildet, dass dessen Außenwandung an einer Innenwandung des Gehäuseteiles anliegt. Es kann jedoch auch vorgesehen sein, dass die der Innenfläche der Seitenwandung des Gehäuseteiles zugewandte Außenwandung des Filters beabstandet von dieser ausgebildet ist, so dass ein Spalt gebildet ist. Der Filter kann jedoch auch derart dimensioniert sein, dass dieser derart in das Gehäuseteil angepasst ist, dass eine Presspassung erfolgt. Durch Betätigung des Sprechventiles erfolgt vorzugsweise über diese Pressung, die einer Teilkomprimierung gleichkommt, keine weitergehende Komprimierung mehr. Bei Vorsehung einer Presspassung kann der Filter durch selbige fixiert werden, so dass er bei der Betätigung des Deckelteils nicht bewegt wird. Dies hängt auch von der Dimensionierung des Aufnahmeraums für den Filter im Gehäuseteil ab, wobei je nach Freiraum zwischen Verschlussteil und Boden des Gehäuseteils dann der Filter gleichwohl dem Stellmittel folgen kann, so die aufgewendete Kraft groß genug ist. Auch hängt dies davon ab, wie groß die auf die Presspassung ausgeübte Kraft ist. Ist Letztere gering, kann der Filter leichter mitbewegt werden.

Das Verschlussteil des Stellmittels kann auf verschiedenste Weise ausgebildet sein. Einerseits muss dieses die Funktion erfüllen, einen hinreichend dichten Verschluss mit dem durch das Gehäuseteil zur Verfügung gestellten Ventilsitz zu erzielen, andererseits muss dieses auch so dimensioniert sein, dass hinreichend Luft über den Filter in das Tracheostoma ein- bzw. ausströmen kann. Hierzu kann beispielsweise vorgesehen sein, dass im Gehäuseteil die proximale Öffnung erheblich kleiner ausgestaltet wird als die distale Öffnung, so dass das Verschlussteil einen geringeren Durchmesser aufweist als z.B. das Deckelteilbefestigungselement. Dann kann das Verschlussteil beispielsweise als plattentellerförmig angesprochen werden, und tritt bei Überführung des Sprechventiles aus der Offenstellung in die Geschlossenstellung mit dessen Randbereich mit dem Ventilsitz, gebildet durch den Boden des Gehäuseteiles und dem dort vorliegenden umlaufenden Rand, in Wirkverbindung, um den Luftstrom zu unterbrechen. Der Filter kann bei dieser Ausgestaltung derart ausgebildet sein, dass dieser mit seiner Außenwandung, zugewandt der Seitenwandung des Gehäuseteiles, das Verschlussteil horizontal überragt, mithin einen größeren Durchmesser aufweist als das Verschlussteil. Je nach Dimensionierung des Sprechventiles kann jedoch auch bei einer Maximierung des Durchmessers des Verschlussteiles und bei Ausbildung desselben als plattentellerförmig ein ausreichender Luftstrom in und aus dem Tracheostoma zur Verfügung gestellt sein, soweit insbesondere ein Spalt zwischen der Außenwandung des Filterkörpers und einer Innenfläche der Seitenwandung und entsprechend zwischen letzterer und dem Verschlussteil zur Verfügung gestellt ist. Soweit das Verschlussteil des Stellelementes mit dem Ventilsitz zur Erzielung einer hinreichend dichtenden Wirkung zusammenwirkt, kann dieses auch in dem den Ventilsitz überragenden, sich horizontal erstreckenden Teilbereich Öffnungen aufweisen, durch welche Luft, die eingeatmet oder ausgeatmet wird, strömen kann. Es kann beispielsweise auch vorgesehen sein, dass sowohl das Verschlussteil als auch das Deckelteilbefestigungselement am distalen Ende des Stellmittels einen geringen Durchmesser aufweisen, d.h. einen Durchmesser, der deutlicher geringer ist als der Innendurchmesser des Gehäuseteiles, wohingegen der Filter einen größeren Durchmesser aufweist, insbesondere einer solchen, der im Wesentlichen dem Innendurchmesser des Gehäuseteiles entspricht oder aber bei Vorsehung einer Presspassung sogar größer ist, so dass eine Anlage des Filterkörpers über dessen Außenwandung an eine Innenfläche der Seitenwandung des Gehäuseteils erfolgt. Die Durchmesser des Deckelbefestigungselementes, soweit vorhanden, als auch des Verschlussteiles müssen nicht, können jedoch unterschiedlich ausgebildet sein.

Das Stellmittel ist vorzugsweise aus einem rigiden, insbesondere unelastischem bzw. biegefesten Material wie Polyethylen oder Polypropylen, insbesondere high-density Polyethylen (HDPE), gefertigt. Insbesondere bei einer einteiligen Ausbildung mit dem Deckelteil kann es auch aus Materialien gefertigt sein, wie diese vorstehend für das Deckelteil beschrieben sind.

Vorteilhafterweise ist das Stellmittel an dem Deckelteil angeordnet. Diese Anordnung kann auf vielfältige Art und Weise erfolgen. Beispielsweise kann bei Nichtvorsehung eines Deckelteilbefestigungselement eine Anordnung des Stellmittels am Deckelteil auch im Wesentlichen über den Filter erfolgen, wobei dann das Deckelteil mit dem Filter durch Verklebung, Verhakung oder auf sonstige Weise verbunden ist. In einer solchen Ausgestaltung ohne ein Deckelbefestigungselement weist das Stellmittel vorteilhafterweise zur Erzielung einer hinreichenden Festigkeit ein Verbindungsteil, stab- oder wandförmig oder sonstiger Art wie bereits vorstehend beschrieben, auf, welches dann insbesondere die angreifenden Kräfte bei Betätigung des Sprechventiles zur Überführung aus einer Offenstellung in eine Geschlossenenstellung auf das Verschlussteil überträgt zur Erzielung einer Wirkverbindung mit dem Ventilsitz, letzterer zur Verfügung gestellt durch das Gehäuseteil.

Bevorzugt ist das Deckelteil mit dem Deckelteilbefestigungselement des Stellmittels verbunden. Dabei kann z.B. das Deckelteil mit seiner proximalen Unterseite (Innenwandung) mit einer distalen Oberfläche des Deckelteilbefestigungselementes verbunden sein. Die Verbindung kann dabei in jeder Form erfolgen, beispielsweise durch Verklebung, einen Klettverschluss, Verschraubung oder Verrastung. Besonders bevorzugt erfolgt eine Verrastung oder aber eine Verklebung. Auch kann vorgesehen sein, dass das Deckelteilbefestigungselement derart mit dem Deckelteil verbunden ist, dass ein Innenrand des Deckelteiles an einer zentralen Öffnung desselben mit einem Außenrand des Deckelbefestigungselementes verbunden ist. Das Deckelteil kann beispielsweise vollständig oder teilweise oberhalb des Deckelteilbefestigungselementes angeordnet sein, so dass dieses nach außen nicht sichtbar ist. Es können Halteelemente, welche von oberhalb des Deckteils durch die Öffnung desselben geführt sind und zumindest teilweise an der Innenwandung des Deckelteils anliegen, vorgesehen sein. Die Halteelemente können dabei beispielsweise federarmartig ausgebildet sein. Dabei können zusätzlich Öffnungen im Deckelteil bzw. im Deckelteilbefestigungselement distal vorgesehen sein zur Erzielung eines Luftdurchtrittes. Einströmöffnungen in der Seitenwandung des Gehäuseteiles müssen dann nicht, können aber vorgesehen sein. Deckelteil und Stellmittel können auch einteilig ausgebildet sein. Dann sind diese bevorzugt im 1-Komponenten-Spritzguß hergestellt. Weiter bevorzugt sind diese aus dem gleichen Material hergestellt, bevorzugt aus einem elastischen Material, aus dem das Deckelteil bevorzugt gebildet ist. Dem Stellmittel kann dann eine höhere Steifheit bzw. eine verminderte Elastizität durch beispielsweise Verdickung der Wandstärken oder sonstige mechanische Mittel wie Verstärkungsrippen, Sicken, Hohlräume o.ä. mitgegeben werden. Auch eine Herstellung im 2-Komponenten-Spritzguß ist möglich, mit Verwendung eines elastischen Materials für das Deckelteil und eines anderen Materials für das Stellmittel, welches dann elastisch oder unelastich bzw. rigid sein kann.

Bevorzugt gemäß der vorliegenden Erfindung umfasst das Stellmittel zumindest das Verschlussteil, bevorzugt auch das Deckelteilbefestigungselement, als auch ein Verbindungsteil des erfindungsgemäßen Sprechventils. Der Verbindungsteil verbindet, wie vorstehend beschrieben, das Deckelteilbefestigungselement oder das Deckelteil mit dem Verschlussteil. Das Stellmittel, genauer dessen Verschlussteil, bildet zusammen mit dem Ventilsitz, zur Verfügung gestellt durch das Gehäuseteil, das Ventilelement des erfindungsgemäßen Sprechventils. Bevorzugt ist das Stellmittel nur mit dem Deckelteil verbunden, und weiter bevorzugt das Deckelteil nur mit dem Gehäuse. Dann bildet das Deckelteil mit dem Stellmittel eine Einheit, die in das Gehäuseteil eingesetzt wird. Der Deckelteil übernimmt dabei die Funktion eines Rückstellmittels und stellt die hinreichende Elastizität zur Schließung des Ventilelementes zur Verfügung. Die Führung des Stellmittels im Inneren des Gehäuseteiles erfolgt über eine Zentrierung durch das Deckelteil, welches bevorzugt über Rastverbindungen mit dem Gehäuseteil verbunden ist. Eine weitergehende Führung kann dann erzielt werden, wenn insbesondere die Außenwandung des Filters an der Innenseite der Seitenwandung des Gehäuseteiles anliegt oder der Filter in einem Presssitz im Gehäuseteil aufgenommen ist. Darüber hinaus kann die Führung auch insbesondere über den Seitenrand des Verschlussteiles erfolgen, so dieses mit seinem Durchmesser in etwa dem Innendurchmesser des Gehäuseteiles angepasst ist.

Diese und weitere Vorteile der vorliegenden Erfindung werden anhand des nachfolgenden Ausführungsbeispiels nähergehend erläutert. Es zeigen:
Fig. 1 ein Sprechventil in lateraler Schnittansicht in einer Offenstellung;
Fig. 2 das Sprechventil gem. Fig. 1 in lateraler Schnittansicht in Geschlossenstellung;
Fig. 3 eine weitere Ausgestaltung des Sprechventiles in lateraler Schnittansicht in Offenstellung; und
Fig. 4 das Sprechventil gemäß Fig. 3 in lateraler Schnittansichtin Geschlossenstellung.

Zunächst sei vorausgeschickt, dass die in den Figuren dargestellten Ausgestaltungen lediglich beispielhaft sind, und den Schutz der vorliegenden Erfindung nicht beschränken sollen. Die dort dargestellten Ausführungen können insbesondere mit den vorstehenden beschriebenen Merkmalen der allgemeinen Beschreibung zur weiteren Ausgestaltung kombiniert werden. So ist insbesondere darauf hinzuweisen, dass die gezeigte Verbindung zwischen Deckelteil und Gehäuseteil als auch zwischen Deckelteil und Stellmittel nur eine der möglichen Ausgestaltungen ist, ebenso wie die dort gezeigte Ausgestaltung des Stellmittels mit angeordnetem Filter und einem Verschlussteil, welche einen Spalt zwischen einer Innenfläche einer Seitenwandung des Gehäuseteiles und einem Seitenrand des Verschlussteiles zeigt. Auch die Form des Deckelteiles ist nur eine der möglichen vielgestaltigen Ausführungsformen desselben, welches insbesondere hut- oder aber kappenartig ausgestaltet sein kann mit einem zervikalen Rand, der der Hautoberfläche eines Benutzers zugewandt ist. Auch kann der proximal angeordnete Ventilsitz nicht durch den Boden des Gehäuses, sondern z.B. durch einen Rücksprung in der Gehäusewandung zur Verfügung gestellt sein, so dass dort eine ringförmige Fläche gebildet ist. Der proximal angeordnete Ventilsitz ist dann zwar weiter vom Körper des Trägers entfernt, gleichwohl aber näher an diesem angeordnet als an eine mindestens eine distale Öffnung des Sprechventils.

Des Weiteren sei darauf verwiesen, dass die in der Figurenbeschreibung angegebene Bezugszeichen den Schutzbereich der vorliegenden Erfindung nicht beschränken, sondern lediglich auf die in den Figuren gezeigten Ausführungsbeispiele verweisen.

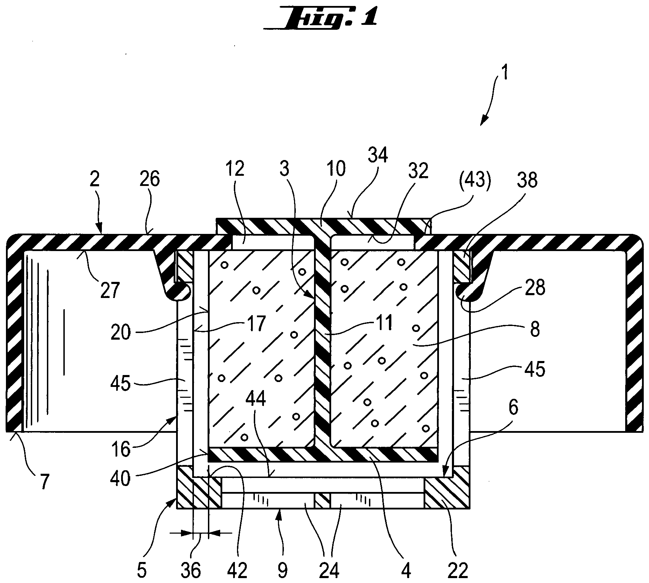

Fig. 1 zeigt ein Sprechventil 1, das auf eine Tracheostomakanüle oder aber ein Tracheostomapflaster aufgesetzt werden kann, welche nicht dargestellt sind. Das Sprechventil 1 weist einen Deckelteil 2, ein Stellmittel 3, einen Gehäuseteil 5 und einen Filter 8 auf. Das Deckelteil 2 überragt das Gehäuseteil 5 lateral und vertikal und weist insbesondere einen zervikalen Rand 7 auf. Es ist im Wesentlichen hut- oder kappenförmig ausgebildet, und wäre in einer Draufsicht als in etwa oval ausgebildet anzusprechen. Das Deckelteil 2 weist eine distale Außenseite 26 und eine distale Unterseite 27 auf. Das Deckelteil ist kappenförmig auf dem Gehäuseteil 5 aufgesetzt und mit diesem über Rastelemente, ausgebildet als Rastnasen 28, verbunden. Diese Rastnasen 28 rasten in einen oberen Rand 38 des Gehäuseteiles 5 und dort vorgesehene Ausnehmungen ein. In der gezeigten Ausführungsform sind die Ausnehmungen gleichzeitig Einströmöffnungen 45. Vorzugsweise sind dabei vier Rastnasen 28 vorgesehen, die an der Unterseite 27 des Deckelteiles 2 angeordnet sind. Das Deckelteil 2 ist vollständig aus einem gummielastischen Material gefertigt. Das Deckelteil 2 weist eine distale Öffnung 12 auf, die zur Aufnahme eines Deckelteilbefestigungselementes 10 des Stellmittels 5 vorgesehen ist. Das Deckelteilbefestigungselement 10 verschließt vorteilhaft die distale Öffnung 12 des Deckelteiles. Das Deckelteilbefestigungselement 10 kann dabei auch Öffnungen aufweisen, die bei Überführung des Sprechventiles 1 aus einer Offenstellung in eine Geschlossenstellung durch z.B. einen Finger des Benutzers verschlossen werden. Das Deckelteilbefestigungselement 10 kann gemäß einer alternativen Ausgestaltung mit seiner Unterseite 32 in einem Randbereich mit der Außenseite 26 des Deckelteiles 2 mittels einer Klebeverbindung 43 klebend fest verbunden sein.

Das Stellmittel 3 weist neben dem Deckelteilbefestigungselement 10 des Weiteren einen Verbindungsteil 11, der rohr- oder stabförmig ausgebildet ist, und das Deckelteilbefestigungselement 10 mit einem Verschlussteil 4, angeordnet nah einer proximalen Öffnung 9 des Gehäuseteiles 5, auf. Das Verbindungsteil 11 erstreckt sich durch den Filter 8, der einteilig ausgebildet ist und eine Außenwandung 20 aufweist, die beabstandet einer Innenfläche 17 einer Seitenwandung 16 des Gehäuseteiles 5 ist. Der Durchmesser des Filters 8 entspricht im Wesentlichen dem Durchmesser des Deckelteilbefestigungselementes 10 und des Verschlussteiles 4, so dass zwischen einem Seitenrand 40 des Verschlussteiles 4 und der Innenfläche 17 der Seitenwandung 16 des Gehäuseteiles 5 ein Spalt 36 ebenso gebildet ist wie zwischen der Außenwandung des Filters 8 und der Innenfläche 17 der Seitenwandung 16 des Gehäuseteils 5.

Das Verschlussteil 4, welches plattentellerförmig ausgebildet ist, wirkt beim Verschließen des Sprechventiles 1 mit einem Ventilsitz 6 zusammen. Der Ventilsitz 6 ist gebildet durch einen Teilbereich eines Bodens 22, der gebildet ist aus einem umlaufenden Rand 44 und einem zur Verstärkung innerhalb der Öffnung 9 angeordneten drei, vier oder mehrarmig ausgebildeten Kreuz aus Verbindungsstreben 24. Eine Oberfläche 42 des umlaufenden Randes 44 bildet teilweise den Ventilsitz 6.

Das Gehäuseteil 5 weist Einströmöffnungen 45 auf, die sich in der Seitenwandung 16 des Gehäuseteiles 5 befinden. Die Einströmöffnungen 45 weisen eine laterale Ausdehnung auf, die in etwa der Höhe des Filters 8 entsprechen. Insbesondere sind die Einströmöffnungen 45 derart in der Seitenwandung 16 des Gehäuseteils 5 angeordnet, dass diese in Offenstellung der Sprechventiles 1 im Wesentlichen oberhalb des Verschlussteiles 4 des Stellmittels 3 angeordnet sind.

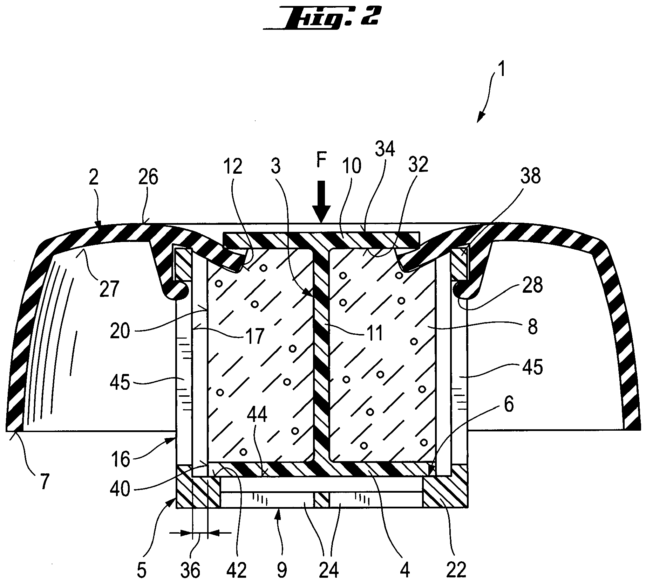

Fig. 2 zeigt das Sprechventil 1 gemäß Fig. 1 in Geschlossenstellung. Zur Betätigung des Sprechventiles 1 wird eine Kraft F auf das Deckelteil 2 bzw. des Deckelteilbefestigungselement 10 in Offenstellung wie in Fig. 1 gezeigt ausgeübt, so dass sich das elastische Deckelteil 2 verformt und das Stellmittel 3 derart im Gehäuseteil 5 bewegt wird, dass das Verschlussteil 4 mit dem Ventilsitz 6 zusammenwirkt und das Sprechventil 1 verschließt. Nach Verschluss des Sprechventils 1 kann die Luft nicht mehr beim Ausatmen durch die Ausströmöffnung 9 nach außen gelangen und wird somit an den Stimmbändern den Benutzers bzw. den künstlichen Stimmbändern des Benutzers vorbeigeführt, um ein Sprechen zu ermöglichen.

Wird die Kraft F, die auf das Deckelteil 2 wirkt, um das Stellmittel 3 zu bestätigen, gelöst, erfolgt eine Rückstellung des Stellmittels 3 bzw. des Verschlussteiles 4. Die Rückstellkräfte werden durch das gummielastische Material des Deckelteils 2 herbeigeführt, gegebenenfalls ergänzt durch die Formgebung des Deckelteils 2 in geometrischer Hinsicht. Material- und Formgebung des Deckelteils 2 sind insbesondere derart ausgestaltet, dass einerseits ein komfortables Verschließen des Sprechventiles 1 erfolgen kann, ohne dass ein zu fester Druck auf das Sprechventil 1 erfolgt, der zu einem Verschieben der Trachealkanüle führen würde, andererseits ist dieser derart eingestellt, dass eine sichere Zurückstellung des Sprechventiles 1 beim Lösen der auf das Deckelteil 2 wirkenden Kraft erfolgt.

Die in Fig. 2 gezeigte Ausführungsform zeigt das Sprechventil 1 aus Fig. 1 ohne die Klebestelle 43. Vielmehr ist das Deckelteil 2 mit dem Deckelteilbefestigungselement 10 klemmend verbunden. Die Klemmwirkung kann einerseits dadurch erreicht sein, dass das Deckelteilbefestigungselement 10 hier nicht dargestellte Laschen aufweist, die sich unter das Deckelteil 2 klemmen. Anderseits kann auch eine Klemmwirkung durch Verklemmen des Deckelteils 2 zwischen Deckelteilbefestigungselement 10 und Filter 8 erfolgen.

In der in den Fig. 1 und 2 gezeigten Ausgestaltung wird der Filter 8 nur minimal durch das Deckelteil 2 in Schließstellung komprimiert. Um eine jegliche Komprimierung des Filters 8 zu vermeiden, kann z.B. alternativ, wie auch vorstehend angesprochen, das Deckelteil 2 derart angeordnet sein, dass es mit seiner Unterseite 27 an einer distalen Oberfläche 34 des Deckelteilbefestigungselementes 10 angeordnet ist, beispielsweise klebend. Die Anordnung kann dabei nur in einem Randbereich des Deckelteils 2 erfolgen, oder aber das Deckelteil 2 kann das gesamte Deckelteilbefestigungselement überragen. Dann kann auch das Deckelteilbefestigungselement in seinem Durchmesser kleiner ausgebildet werden, oder gar entfallen, so dass das Deckelteil 2 mit seiner Unterseite 27 mit dem Verbindungsteil 11 des Stellmittels 5 verbunden ist.

In einer weiteren alternativen Ausführungsform kann insbesondere vorgesehen sein, dass die proximale Öffnung 9 einen geringeren Durchmesser aufweist, so dass entsprechend auch das Verschlussteil 4 einen geringen Durchmesser aufweist. Der Filterkörper 20 hingegen kann seinen Durchmesser wie in den Figuren 1 und 2 gezeigt beibehalten, so dass dieser über einen Seitenrand 40 des Verschlussteiles 4 übersteht, und hierdurch eine sehr gute Feuchte- und Wärmetauscherfunktion zur Verfügung stellt. Entsprechend wäre in einer solchen Ausführungsform der Boden 22 mit einem breiteren umlaufenden Rand 44 ausgebildet, und gegebenenfalls könnten dann auch die Verstärkungsstreben 24 innerhalb der proximalen Öffnung 9 weggelassen werden.

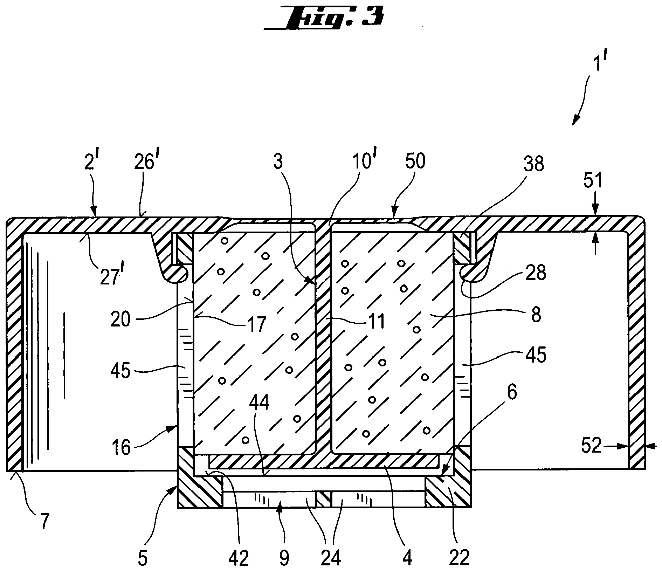

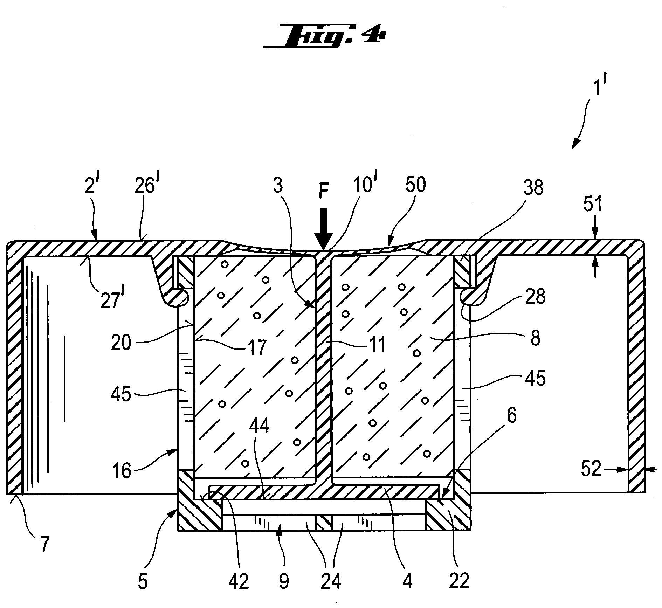

Fig. 3 zeigt eine weitere Ausgestaltung des Sprechventils in lateraler Schnittansicht in Offenstellung. Das Sprechventil 1' unterscheidet sich von der Ausgestaltung aus Fig. 1 und 2 dadurch, dass das Deckelteil 2' und das Stellmittel 3 einteilig ausgebildet sind. Das Deckelteil 2' geht im Bereich von 10' in das Stellmittel 3 über. Insbesondere ist zur vereinfachten Handhabung des Sprechventils 1' die Wandstärke des Deckelteils 2' im Bereich des Übergangs 10' vermindert. Vorzugsweise sind Stellmittel 3 und Deckelteil 2' aus einem Material gefertigt. Dieses ist insbesondere semirigid, das heißt das Material weist eine ausreichende Stabilität auf, um beispielsweise das Verschlussteil 4 mit angemessenem Druck auf den Ventilsitz 6 zu drücken, ohne dass das Verbindungsteil 11 dabei knickt. Weiterhin ist das Material so elastisch, dass eine ausreichende Verformung durch eine von einem Benutzer ausgeübte Kraft F, die in Fig. 4 dargestellt ist, das Deckelteil 2' an der materialverringerten Stelle einbeult, so dass das Stellmittel 3 das Verschlussteil 4 in eine Geschlossenstellung gemäß Fig. 4 bewegt. Insbesondere ist der Übergangsbereich 10' als Membran 50 ausgebildet, so dass das Material mit einem geringen Kraftaufwand 11 eingedellt werden kann. Weiterhin ist das Material derart elastisch, dass dieses eine Rückstellkraft auf das Stellmittel ausübt, die eine selbstständige Rückstellung des Verschlussteiles 4 in eine Offenstellung ermöglicht. Vorzugsweise umfasst das Material ein medizinisches Silikon.

Das Deckelteil 2' weist weiterhin eine distale Wanddicke 51 auf, die sich von einer zervikalen Wanddicke 52 unterscheidet. Insbesondere ist die distale Wanddicke 51 des Deckelteils 2' größer als die zervikale Wanddicke 52. Es wird über an einer Unterseite 27' angeordnete Rastnasen 28 wie bei der ersten Ausführungsform mit dem Gehäuse 5 verbunden.

Fig. 4 zeigt das Sprechventil aus Fig. 3 in lateraler Schnittansicht in der betätigten Geschlossenstellung. Auf die Membran 50 wird eine Kraft F aufgewendet, die die Membran 50 eindellt und somit das Verbindungsteil 11 beziehungsweise das Verschlussteil 4 nach proximal verschiebt. In der gezeigten Ausführung ist zu sehen, dass sich das Verbindungsteil 11 relativ zum Filter 8 bewegt, wobei sich auch der Filter 8 bewegen kann. Dies wird insbesondere dadurch erreicht, dass der Filter 8 passgenau beziehungsweise mit einer Übermaßpassung im Gehäuse 5 angeordnet ist. Das Verbindungsteil 11 bewegt sich durch die Öffnung des Filters 8 und ist nicht mit diesem verbunden. Durch die gezeigte Ausgestaltung wird verhindert, dass eine starke Komprimierung des Filters 8 durch die Betätigung des Sprechventils 1' erfolgt. Hierzu ist insbesondere auch vorgesehen, dass distal des Filters 8 ein Freiraum zwischen Filter 8 und Deckelteil 2' vorgesehen ist, der eine Ausdehnung aufweist, die größer oder gleich dem Bewegungsspielraum des Ventilsitzes 6 ist. Ist der Abstand bzw. Freiraum zwischen Verschlussteil 4 und Rand 44 größer, würde unter Erhöhung der Kraft F der Filter 8 der Bewegung des Stellmittels 3 folgen unter gegebenenfalls geringer Komprimierung.

Die Anbringung des Deckelteiles 2' am oberen Rand 38 des Gehäuseteils 5 erfolgt analog der Ausgestaltung des Sprechventils aus Fig. 1 und 2.

Durch die vorliegende Erfindung wird ein äußerst einfach aufgebautes Sprechventil zur Verfügung gestellt, welche aus nur drei oder vier Teilen, die einfach aufgebaut sind, hergestellt werden kann, nämlich einem Filter, einem Stellmittel, einem Deckelteil (gegebenenfalls einteilig mit dem Stellmittel) und einem Gehäuseteil. Die für eine Rückstellung notwendige Elastizität wird durch das Deckelteil zur Verfügung gestellt, Federelemente oder ähnliches fehlen vollständig. Zudem kann das erfindungsgemäße Sprechventil einige weitere Vorteile in sich vereinen aufgrund der vielfältigen Ausgestaltungsmöglichkeiten des Deckelteiles im Hinblick auf eine Vorwärmung der Luft als auch im Hinblick auf eine Nichtkomprimierung des Filterkörpers, um ein Austreten größerer Mengen an Feuchtigkeit aus diesem zu vermeiden.

## Patentansprüche

1. Sprechventil (1) für Laryngektomierte oder Tracheotomierte mit einem Deckelteil (2), einem Stellmittel (3), einem Gehäuseteil (5) und einem Filter (8), wobei das Gehäuseteil (5) einen proximal in diesem angeordneten Ventilsitz zur Verfügung stellt, wobei das Stellmittel (3) ein Verschlussteil (4) umfasst und wobei das Verschlussteil (4) bei Überführung des Sprechventils (1) aus einer Offenstellung in eine Geschlossenstellung mit dem Ventilsitz zusammenwirkt, wobei das Deckelteil (2) zumindest teilweise aus einem elastischen Material gebildet ist, wobei durch eine Überführung des Sprechventiles aus der Offenstellung in die Geschlossenstellung eine zumindest teilweise Verformung zumindest eines Bereiches des Deckelteiles (2) erfolgt, und wobei der Filter (8) am Stellmittel (3) derart angeordnet ist, dass der Filter (8) der Bewegung des Stellmittels (3) im Wesentlichen folgen kann.

2. Sprechventil (1) nach Anspruch 1, **dadurch gekennzeichnet, dass** der Filter (8) bei Betätigung des Sprechventils (1) im Wesentlichen unkomprimiert bleibt.

3. Sprechventil (1) nach einem der vorhergehenden Ansprüche, **dadurch gekennzeichnet, dass** das Stellmittel (3) an dem Deckelteil (2) angeordnet ist.

4. Sprechventil (1) nach einem der vorhergehenden Ansprüche, **dadurch gekennzeichnet, dass** zumindest ein elastisch verformbarer Bereich des Deckelteils (2) als Rückstellmittel zur Überführung aus der Geschlossenstellung in die Offenstellung fungiert.

5. Sprechventil (1) nach einem der vorhergehenden Ansprüche, **dadurch gekennzeichnet, dass** das elastische Material des Deckelteils (2) ein gummielastisches Material ist.

6. Sprechventil nach einem der Ansprüche 1 bis 5, **dadurch gekennzeichnet, dass** das elastische Material des Deckelteils (2) ein linear-elastisches Material ist.

7. Sprechventil (1) nach einem der vorhergehenden Ansprüche, **dadurch gekennzeichnet, dass** das Deckelteil (2) aus dem elastischen Material besteht.

8. Sprechventil (1) nach einem der vorhergehenden Ansprüche, **dadurch gekennzeichnet, dass** das Deckelteil (2) eine Seitenwandung (16) das Gehäuseteils (5) zumindest teilweise überragt.

9. Sprechventil (1) nach einem der vorhergehenden Ansprüche, **dadurch gekennzeichnet, dass** das Deckelteil (2) nach distal eine Höhe umfasst, die mindestens 30 % einer Höhe des Gehäuseteils (5) entspricht.

10. Sprechventil (1) nach einem der vorhergehenden Ansprüche, **dadurch gekennzeichnet, dass** das Deckelteil (2) das Gehäuseteil (5) lateral und vertikal überragt.

11. Sprechventil (1) nach einem der vorhergehenden Ansprüche, **dadurch gekennzeichnet, dass** der Deckelteil (2) einen zervikalen Rand (7) umfasst.

12. Sprechventil (1) nach einem der vorhergehenden Ansprüche, **dadurch gekennzeichnet, dass** das Deckelteil (2) lösbar mit dem Gehäuseteil (5) und/oder Stellmittel (3) verbunden ist.

13. Sprechventil (1) nach einem der vorhergehenden Ansprüche, **dadurch gekennzeichnet, dass** das Deckelteil (2) mittels einer Rastverbindung an dem Gehäuseteil (5) angeordnet ist.

14. Sprechventil (1) nach einem der vorhergehenden Ansprüche, **dadurch gekennzeichnet, dass** das Gehäuseteil (5) zumindest eine Einströmöffnung (45) in der Seitenwandung (16) umfasst.

15. Sprechventil (1) nach Anspruch 14, **dadurch gekennzeichnet, dass** die zumindest eine Einströmöffnung (45) eine laterale Ausdehnung bis zu einer Höhe des Filters (8) aufweist.

16. Sprechventil (1) nach einem der vorhergehenden Ansprüche, **dadurch gekennzeichnet, dass** das Gehäuseteil (5) und/oder das Stellmittel (3) aus einem unelastischen Material bestehen.

17. Sprechventil (1) nach einem der vorhergehenden Ansprüche, **dadurch gekennzeichnet, dass** das Stellmittel (3) zumindest das Verschlussteil (4), ein Deckelteilbefestigungselement (10) und ein Verbindungssteil (11) umfasst.

## Claims

1. Speaking valve (1) for laryngectomy or tracheotomy patients having a cover part (2), a control means (3), a housing part (5) and a filter (8), wherein the housing part (5) provides a valve seat, which is proximally arranged in the housing part (5), wherein the control means (3) comprises a closure part (4) and wherein the closure part (4) cooperates with the valve seat when the speaking valve (1) is moved from an open position into a closed position, wherein the cover part (2) is formed at least partially from an elastic material, wherein an at least partial deformation takes place at least in a region of the cover part (2) when the speaking valve is moved from an open position into a closed position, and wherein the filter (8) is arranged at the control means (3) in such a way that the filter(8) can follow essentially the movement of the control means (3).

2. Speaking valve (1) according to claim 1, **characterized in that** the filter (8) remains essentially uncompressed when the speaking valve is activated.

3. Speaking valve (1) according to anyone of the preceding claims, **characterized in that** the control means (3) is arranged at the cover part (2).

4. Speaking valve (1) according to anyone of the preceding claims, **characterized in that** at least one elastically deformable region of the cover part (2) functions as a resetting means for performing a movement from the closed position into the open position.

5. Speaking valve (1) according to anyone of the preceding claims, **characterized in that** the elastic material of the cover part (2) consists of a rubber-elastic material.

6. Speaking valve (1) according to anyone of the claims 1 to 5, **characterized in that** the elastic material of the cover part (2) consists of a linear-elastic material.

7. Speaking valve (1) according to anyone of the preceding claims, **characterized in that** the cover part (2) consists of an elastic material.

8. Speaking valve (1) according to anyone of the preceding claims, **characterized in that** the cover part (2) overlaps at least partially a sidewall (16) of the housing part (5).

9. Speaking valve (1) according to anyone of the preceding claims, **characterized in that** the cover part (2) comprises a height in distal direction which corresponds to at least 30% of a height of the housing part (5).

10. Speaking valve (1) according to anyone of the preceding claims, **characterized in that** the cover part (2) laterally and vertically overlaps the housing part (5).

11. Speaking valve (1) according to anyone of the preceding claims, **characterized in that** the cover part (2) has a cervical edge (7).

12. Speaking valve (1) according to anyone of the preceding claims, **characterized in that** the cover part (2) is detachably connected with the housing part (5) and/or the control means (3).

13. Speaking valve (1) according to anyone of the preceding claims, **characterized in that** the cover part (2) is attached to the housing part (5) by means of a snap-on connection.

14. Speaking valve (1) according to anyone of the preceding claims, **characterized in that** the housing part (5) comprises at least one inflow opening (45) in the sidewall (16).

15. Speaking valve (1) according to claim 14, **characterized in that** the at least one inflow opening (45) has a lateral expansion up to a height of the filter (8).

16. Speaking valve (1) according to anyone of the preceding claims, **characterized in that** the housing part (5) and/or the control means (3) consist of an inflexible material.

17. Speaking valve (1) according to anyone of the preceding claims, **characterized in that** the control means (3) comprises at least the closure part (4), an attachment element (10) for the cover part (2) and a connecting element (11).

## Revendications

1. Valve de phonation (1) pour laryngectomisés ou trachéotomisés avec une partie couvercle (2), un moyen de réglage (3), une partie boîtier (5) et un filtre (8), dans lequel ladite partie boîtier (5) présente un siège de valve disposé à proximité dans ladite partie boîtier (5), dans laquelle ledit moyen de réglage (3) comprend un élément de fermeture (4) et dans laquelle ledit élément de fermeture (4) coopère avec ledit siège de valve lorsque ladite valve de phonation (1) est transférée d'une position ouverte à une position fermée, ladite partie couvercle (2) étant formée au moins partiellement en un matériau élastique, dans laquelle une déformation au moins partielle d'au moins une région de ladite partie couvercle (2) a lieu par un transfert de ladite valve de phonation de la position ouverte à la position fermée, et dans laquelle ledit filtre (8) est disposé sur ledit moyen de réglage (3) de telle sorte que ledit filtre (8) puisse suivre essentiellement le mouvement dudit moyen de réglage (3).

2. Valve de phonation (1) selon la revendication 1, **caractérisée en ce que** ledit filtre (8) reste essentiellement non comprimé lors de l'activation ladite valve de phonation (1).

3. Valve de phonation (1) selon l'une des revendications précédentes, **caractérisée en ce que** ledit moyen de réglage (3) est disposé sur ladite partie couvercle (2).

4. Valve de phonation (1) selon l'une des revendications précédentes, **caractérisée en ce qu'**au moins une zone élastiquement déformable de ladite partie couvercle (2) sert de moyen de retour pour le transfert de la position fermée à la position ouverte.

5. Valve de phonation (1) selon l'une des revendications précédentes, **caractérisée en ce que** ledit matériau élastique de ladite partie couvercle (2) est un matériau élastique en caoutchouc.

6. Valve de phonation selon l'une des revendications 1 à 5, **caractérisée en ce que** ledit matériau élastique de ladite partie couvercle (2) est un matériau élastique linéaire.

7. Valve de phonation (1) selon l'une des revendications précédentes, **caractérisée en ce que** ladite partie couvercle (2) est constituée dudit matériau élastique.

8. Valve de phonation (1) selon l'une des revendications précédentes, **caractérisée en ce que** ladite partie couvercle (2) dépasse au moins partiellement d'une paroi latérale (16) de ladite partie boîtier (5).

9. Valve de phonation (1) selon l'une des revendications précédentes, **caractérisée en ce que** ladite partie couvercle (2) comprend par ailleurs une hauteur qui correspond à au moins 30% d'une hauteur de ladite partie boîtier (5).

10. Valve de phonation (1) selon l'une des revendications précédentes, **caractérisée en ce que** ladite partie couvercle (2) dépasse latéralement et verticalement de ladite partie boîtier (5).

11. Valve de phonation (1) selon l'une des revendications précédentes, **caractérisée en ce que** ladite partie couvercle (2) comporte un bord cervical (7).

12. Valve de phonation (1) selon l'une des revendications précédentes, **caractérisée en ce que** ladite partie couvercle (2) est reliée de manière amovible à ladite partie boîtier (5) et/ou auxdits moyens de réglage (3).

13. Valve de phonation (1) selon l'une des revendications précédentes, **caractérisée en ce que** ladite partie couvercle (2) est montée sur ladite partie boîtier (5) au moyen d'une fixation par encliquetage.

14. Valve de phonation (1) selon l'une des revendications précédentes, **caractérisée en ce que** ladite partie boîtier (5) comporte au moins un orifice d'admission (45) dans ladite paroi latérale (16).

15. Valve de phonation (1) selon la revendication 14, **caractérisée en ce que** l'au moins un orifice d'admission (45) a une extension latérale jusqu'à une hauteur dudit filtre (8).

16. Valve de phonation (1) selon l'une des revendications précédentes, **caractérisée en ce que** ladite partie boîtier (5) et/ou ledit moyen de réglage (3) sont constitués d'un matériau inélastique.

17. Valve de phonation (1) selon l'une des revendications précédentes, **caractérisée en ce que** ledit moyen de réglage (3) comprend au moins un élément de fermeture (4), un élément de fixation (10) de ladite partie couvercle et une partie de liaison (11).
